# EUROPEAN PATENT APPLICATION

(11) **EP 4 629 684 A1**
(43) Date of publication of application: **08.10.2025**
(21) Application number: 25156903.4
(22) Date of filing: 10.02.2025
(51) Int. Cl.: H04W 12/50, H04W 4/80, A61B 5/145, H04W 12/06

(54) **ANALYTE MONITORING DEVICE AND CONTROLLING METHOD THEREOF**

(30) Priority: 04.04.2024 KR 20240045941
(71) Applicant: I-SENS, INC., Seoul 06646 (KR)
(72) Inventor: Park, Jeong Je, 06646 Seoul (KR)
(74) Representative: Westphal, Mussgnug & Partner Patentanwälte mbB

(57) **Abstract**

An analyte monitoring device is disclosed. The analyte monitoring device comprises an analyte sensor configured to be at least partially inserted into a body of a user to detect a signal related to glucose concentration, and a sensor electronics unit electrically connected to the analyte sensor to acquire the signal, wherein the sensor electronics unit is configured to: perform communication connection with a user terminal, transmit information about the sensor electronics unit to the user terminal, receive, from the user terminal, a second security key generated based on the information about the sensor electronics unit and a first security key, perform verification of the second security key, and determine whether to maintain the communication connection based on the verification result.

## Description

### CROSS-REFERENCE TO RELATED APPLICATIONS

This application claims priority from Korean Patent Application No. 10-2024-0045941 filed in the Korean Intellectual Property Office on April 4, 2024, the disclosures of which are incorporated herein by reference.

### BACKGROUND

### 1. Technical Field

The present disclosure relates to an analyte monitoring device, and more particularly, to a continuous glucose monitoring (CGM) device and a controlling method thereof.

### 2. Related Art

Continuous glucose monitoring systems (CGMSs) are systems that acquire blood sugar concentrations of users using sensors in contact with body fluids (e.g., interstitial fluid) of the users and provide the acquired blood sugar concentrations to the users. A CGM system includes CGM device attached to the body of a user to sense signals from the body fluid of the user, and a user terminal device that provides the blood sugar concentration to the user.

The user terminal performs a communication connection with a continuous glucose monitor using a glucose monitoring app that processes glucose signals and receives glucose signals from the continuous glucose monitor. On the other hand, to provide reliable glucose information to the user, it is necessary to manage so that only verified glucose monitoring apps can receive glucose signals from the continuous glucose monitor. For example, a glucose monitoring app may be developed by a business other than the manufacturer of the continuous glucose monitor, which is sometimes referred to as a "third-party app'. If an unauthorized third-party app provides inaccurate glucose values to the user, it could pose a risk to the user.

Therefore, technology is needed to verify glucose monitoring apps.

### SUMMARY

The present disclosure provides an analyte monitoring device that performs verification for an analyte monitoring app and maintains a communication connection with the verified analyte monitoring app.

Objects of the present disclosure are not limited to the above-mentioned objects. That is, other objects that are not described may be obviously understood by those skilled in the art to which the present disclosure pertains from the following description.

According to an embodiment of the present disclosure, there is provided an analyte monitoring device, comprising: an analyte sensor configured to be at least partially inserted into a body of a user to detect a signal related to glucose concentration; and a sensor electronics unit electrically connected to the analyte sensor to acquire the signal, wherein the sensor electronics unit is configured to: perform communication connection with a user terminal, transmit information about the sensor electronics unit to the user terminal, receive, from the user terminal, a second security key generated based on the information about the sensor electronics unit and a first security key, perform verification of the second security key, and determine whether to maintain the communication connection based on the verification result.

The first security key may be assigned to an application authorized by a manufacturer of the analyte monitoring device, and wherein the second security key may be used for verification of the application.

The second security key may include information indicating characteristics of the user terminal or characteristics of the application, and wherein the sensor electronics unit may be configured to operate based on the characteristics of the user terminal or the characteristics of the application when the verification is successful.

The application may be third-party application.

The communication connection may be established through Bluetooth pairing, wherein the information about the sensor electronics unit is transmitted in an unencrypted state before the Bluetooth pairing, and wherein the second security key is received in an encrypted state after the Bluetooth pairing.

The communication connection may be performed based on at least one of Bluetooth and Bluetooth Low Energy (BLE) connection methods.

The sensor electronics unit may perform verification of the second security key by determining whether the second security key matches a pre-stored key.

The sensor electronics unit may obtain a new key by decrypting the second security key based on a predetermined algorithm, and determine whether the new key matches the pre-stored key.

The sensor electronics unit may store a whitelist including a plurality of security keys assigned to a plurality of third parties, including the pre-stored key.

The sensor electronics unit may maintain the communication connection when the verification is successful, and disconnect the communication connection when the verification fails.

According to an embodiment of the present disclosure, there is provided a method for controlling an analyte monitoring device comprising an analyte sensor configured to be at least partially inserted into a body of a user to detect a signal related to glucose concentration and a sensor electronics unit electrically connected to the analyte sensor to acquire the signal, the method comprising: performing communication connection with a user terminal; transmitting information about the sensor electronics unit to the user terminal; receiving, from the user terminal, a second security key generated based on the information about the sensor electronics unit and a first security key; performing verification of the second security key; and determining whether to maintain the communication connection based on the verification result.

The second security key may include information indicating characteristics of the user terminal or characteristics of the application, and wherein the method may further comprise operating based on the characteristics of the user terminal or the characteristics of the application when the verification is successful.

The performing verification of the second security key may comprise: obtaining a new key by decrypting the second security key based on a predetermined algorithm; and determining whether the new key matches the pre-stored key.

Technical solutions of the present disclosure are not limited to the above-mentioned solutions, and solutions that are not mentioned will be clearly understood by those skilled in the art to which the present disclosure pertains from the present specification and the accompanying drawings.

### BRIEF DESCRIPTION OF DRAWINGS

Aspects, features, and advantages of specific embodiments of the present disclosure will become more apparent from the following description with reference to the accompanying drawings:
FIG. 1 is a schematic diagram illustrating a continuous glucose monitoring system according to an embodiment of the present disclosure.
FIG. 2 is a flowchart illustrating a method for verifying a user terminal according to an embodiment of the present disclosure.
FIG. 3 is a schematic diagram for explaining a security key verification method according to an embodiment of the present disclosure.
FIG. 4 is a schematic diagram for explaining operations of the sensor electronics unit according to an embodiment of the present disclosure.
FIG. 5 is a block diagram illustrating a configuration of the analyte monitoring system according to an embodiment of the present disclosure.

### DESCRIPTION OF EXAMPLE EMBODIMENTS

After terms used in the present specification are briefly described, the present disclosure will be described in detail.

General terms that are currently widely used were selected as terms used in embodiments of the present disclosure in consideration of functions in the present disclosure, but may be changed according to the intention of those skilled in the art or a judicial precedent, the emergence of a new technique, and the like. In addition, in a specific case, terms arbitrarily chosen by an applicant may exist. In this case, the meaning of such terms will be mentioned in detail in a corresponding description portion of the present disclosure. Therefore, the terms used in the present disclosure should be defined on the basis of the meaning of the terms and the contents throughout the present disclosure rather than simple names of the terms.

The present disclosure may be variously modified and have several embodiments, and therefore specific embodiments of the present disclosure will be illustrated in the drawings and be described in detail in the detailed description. However, it is to be understood that the present disclosure is not limited to specific exemplary embodiments, but includes all modifications, equivalents, and substitutions without departing from the scope and spirit of the present disclosure. When it is determined that a detailed description of the known art related to the present disclosure may obscure the gist of the present disclosure, the detailed description will be omitted.

The terms "first," "second," and the like may be used to describe various components, but the components are not to be construed as being limited by these terms. The terms are used only to distinguish one component from another component.

Singular forms are intended to include plural forms unless the context clearly indicates otherwise. It should be understood that terms "include" or "comprise" used in the present specification, specify the presence of features, numerals, steps, operations, components, parts mentioned in the present specification, or combinations thereof, but do not preclude the presence or addition of one or more other features, numerals, steps, operations, components, parts, or combinations thereof.

Hereinafter, embodiments of the present disclosure will be described in detail with reference to the accompanying drawings so that those skilled in the art to which the present disclosure pertains may easily practice the present disclosure. However, the present disclosure may be modified in various different forms, and is not limited to embodiments described herein. In addition, in the drawings, portions unrelated to the description will be omitted to obviously describe the present disclosure, and similar reference numerals will be used to describe similar portions throughout the specification.

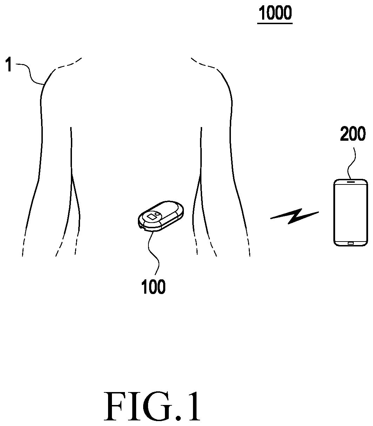

FIG. 1 is a schematic diagram illustrating a continuous glucose monitoring system according to an embodiment of the present disclosure.

Referring to FIG. 1, the analyte monitoring system 1000 may include an analyte monitoring device 100 and an electronic apparatus 200. For example, the analyte monitoring system 1000 may be a Continuous Glucose Monitoring System (CGMS).

The analyte monitoring device 100 may acquire information related to the concentration of an analyte in a user's (1) body fluid. The analyte may include glucose and ketone. The information related to the analyte concentration may be a signal measured by the analyte monitoring device 100 or a concentration value derived from the signal. The analyte monitoring device 100 may be a Continuous Glucose Monitor (CGM). The continuous glucose monitor may include an analyte sensor that is inserted into the user's (1) body and a sensor electronics unit that transmits the signal measured by the sensor to the electronic apparatus 200.

The electronic apparatus 200 may be a user terminal that provides information related to the analyte concentration acquired through the analyte monitoring device 100 to the user (1). For example, the electronic apparatus 200 may display a chart indicating glucose concentration. Additionally, when there is an abnormality in glucose concentration, the electronic apparatus 200 may output an alert. The electronic apparatus 200 may be a smartphone, tablet PC, smartwatch, PDA, or a dedicated receiver.

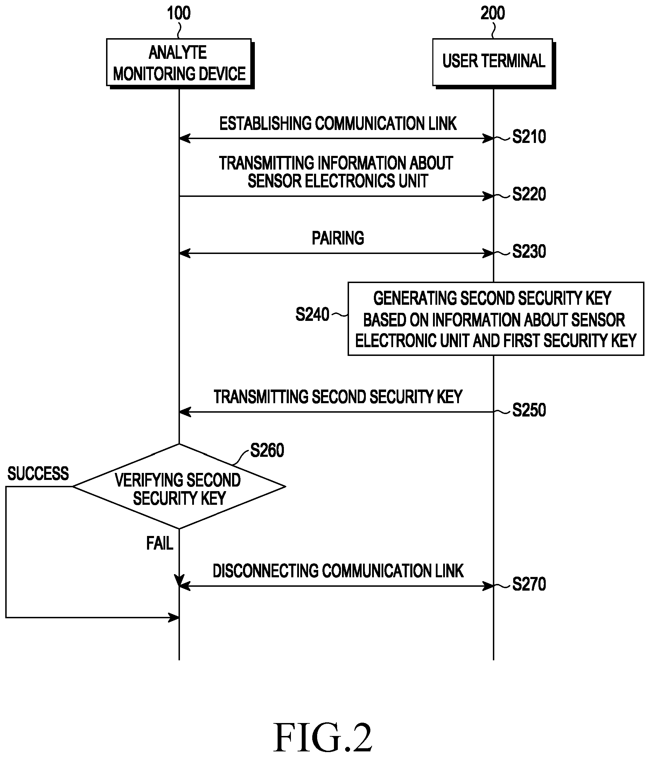

FIG. 2 is a flowchart illustrating a method for verifying a user terminal according to an embodiment of the present disclosure.

Referring to FIG. 2, the analyte monitoring device 100 may perform communication connection with a user terminal 200. The step of performing communication connection may include a step of establishing a communication link between the analyte monitoring device 100 and the user terminal 200 (S210) and a step of pairing with the user terminal (S230). For example, the communication connection may be performed based on Bluetooth or Bluetooth Low Energy (BLE). Step S210 may include a step where the analyte monitoring device 100 transmits an advertising packet to the user terminal 200. Through step S210, the analyte monitoring device 100 may enter a state where it can exchange data with the user terminal 200.

The analyte monitoring device 100 may transmit information about the sensor electronics unit to the user terminal 200 (S220). For example, the information about the sensor electronics unit may include a serial number of the sensor electronics unit. Additionally, the information about the sensor electronics unit may include information indicating the version or manufacturing country of the sensor electronics unit. Meanwhile, the information about the sensor electronics unit may be transmitted to the user terminal 200 in an unencrypted state.

The analyte monitoring device 100 may perform pairing with the user terminal 200 (S230). For example, the analyte monitoring device 100 may perform pairing according to the Bluetooth Low Energy protocol. Accordingly, an encrypted communication channel may be established between the analyte monitoring device 100 and the user terminal 200.

The user terminal 200 may generate a second security key based on the information about the sensor electronics unit and a first security key (S240). The user terminal 200 may generate a new key by combining the information about the sensor electronics unit and the first security key. The user terminal 200 may generate the second security key by encrypting the new key using a predetermined algorithm. **In** one embodiment, the predetermined algorithm may be a symmetric key algorithm such as Advanced Encryption Standard (AES). Meanwhile, the aforementioned steps S210, S220, and S230, and step S240 may be performed by an analyte monitoring app installed on the user terminal 200.

The first security key may be assigned only to third parties authorized by the manufacturer of the analyte monitoring device 100. The third-party app provided by a third party authorized by the manufacturer (i.e., authorized third-party app) may include information about the first security key. The third-party app provided by an unauthorized third party (i.e., unauthorized third-party app) may not include information about the first security key. As will be described later, the first security key may be used to determine whether the analyte monitoring app installed on the user terminal 200 has authorization to connect with the analyte monitoring device 100. The first security key may be stored in the analyte monitoring device 100 upon installation of the analyte monitoring app.

Meanwhile, according to another embodiment, step S240 may also be performed before step S230. Also, although not shown, before step S240, the analyte monitoring device 100 may request a security key from the user terminal 200. **In** response to this, the user terminal 200 may perform S240.

The user terminal 200 may transmit the second security key to the analyte monitoring device 100 (S250). Although not shown, step S250 may be performed in response to a security key request from the analyte monitoring device 100.

The analyte monitoring device 100 may perform verification of the second security key (S260). The analyte monitoring device 100 may determine whether the second security key matches a pre-stored key. **In** one embodiment, the analyte monitoring device 100 may determine whether the second security key is identical to the pre-stored key. If the second security key is identical to the pre-stored key, the analyte monitoring device 100 may determine that the verification of the second security key is successful. If the second security key is different from the pre-stored key, the analyte monitoring device 100 may determine that the verification of the second security key has failed.

In another embodiment, the analyte monitoring device 100 may obtain a new key by decrypting the second security key using a predetermined algorithm. Then, the analyte monitoring device 100 may determine whether the new key is identical to the pre-stored key. If the new key is identical to the pre-stored key, the analyte monitoring device 100 may determine that the verification of the second security key is successful. If the new key is different from the pre-stored key, the analyte monitoring device 100 may determine that the verification of the second security key has failed.

In yet another embodiment, the analyte monitoring device 100 may determine whether the first security key obtained from the new key is identical to the pre-stored key. If the first security key is identical to the pre-stored key, the analyte monitoring device 100 may determine that the verification of the second security key is successful. If the first security key is different from the pre-stored key, the analyte monitoring device 100 may determine that the verification of the second security key has failed.

When the second security key verification is successful (S260: Success), the analyte monitoring device 100 may maintain the communication connection with the user terminal 200. Accordingly, the analyte monitoring device 100 may perform communication with the user terminal 200 according to a predetermined protocol. Additionally, the user terminal 200 may output a message indicating successful verification.

When the second security key verification fails (S260: Fail), the analyte monitoring device 100 may disconnect the communication link with the user terminal 200 (S270). Accordingly, the user terminal 200 cannot receive information about the user's glucose from the analyte monitoring device 100. Additionally, the user terminal 200 may output a message indicating verification failure.

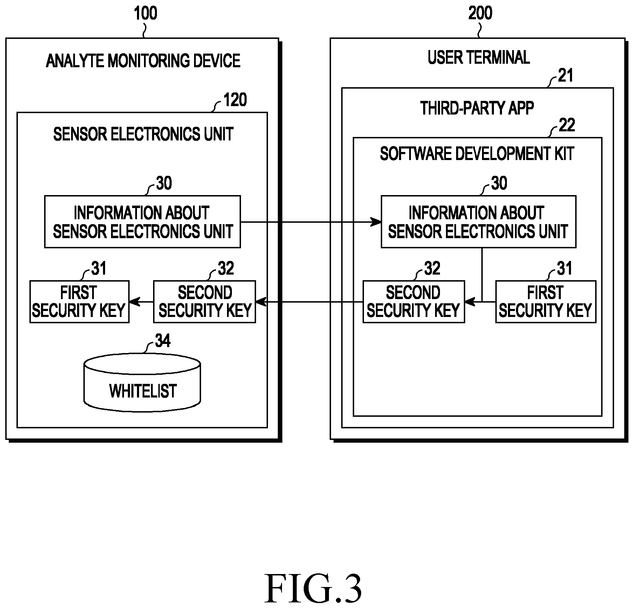

FIG. 3 is a schematic diagram for explaining a security key verification method according to an embodiment of the present disclosure.

Referring to FIG. 3, the analyte monitoring device 100 may transmit information about the sensor electronics unit 30 to the user terminal 200. For example, the information about the sensor electronics unit 30 may be a serial number of the sensor electronics unit 120. The information about the sensor electronics unit 30 may be stored in the sensor electronics unit 120 during the manufacturing stage of the sensor electronics unit 120. The information about the sensor electronics unit 30 may be delivered to a software development kit (software development kit, SDK) 22.

The software development kit 22 may be included in a third-party app 21 installed on the user terminal 200. The third-party app 21 may be developed by a third party other than the manufacturer of the analyte monitoring device 100. The software development kit 22 may include instructions for verifying the third-party app 21. **In** one embodiment, the software development kit 22 may include instructions for generating the second security key 32 based on the information about the sensor electronics unit 30 and the first security key 31. The first security key 31 may be provided by the manufacturer of the analyte monitoring device 100. The third-party app 21 may generate the second security key 32 by executing the instructions included in the software development kit 22.

Meanwhile, the third-party app 21 may provide glucose-related information to the user **1. In** this disclosure, the third-party app 21 may be referred to as an analyte monitoring app. The third-party app 21 may acquire glucose-related information using the software development kit 22 and provide it to the user. The software development kit 22 may include instructions for processing glucose signals. For example, the software development kit 22 may include instructions for calculating blood glucose values based on glucose signals.

The user terminal 200 may transmit the second security key 32 to the analyte monitoring device 100. Specifically, the third-party app 21 may transmit the second security key 32 to the sensor electronics unit 120.

The sensor electronics unit 120 may perform verification of the third-party app 21 based on the second security key 32 and a whitelist 34. The whitelist 34 may store security keys assigned to authorized third parties. The sensor electronics unit 120 may determine whether a key matching the second security key 32 exists in the whitelist 34.

**In** one embodiment, the sensor electronics unit 120 may compare the second security key 32 with the whitelist 34. **In** this case, the whitelist 34 may store encrypted keys generated based on security keys assigned to third parties. **In** this case, to prevent interception by third parties, the second security key 32 may be transmitted to the sensor electronics unit 120 after the pairing between the analyte monitoring device 100 and the user terminal 200 is completed.

In another embodiment, the sensor electronics unit 120 may compare the first security key 31 obtained from the second security key 32 with the whitelist 34. The sensor electronics unit 120 may obtain the first security key 31 by performing decryption of the second security key 32. At this time, the sensor electronics unit 120 may decrypt the second security key 32 using the same algorithm used in generating the second security key 32. The encryption/decryption algorithm may be shared only between the manufacturer of the analyte monitoring device 100 and authorized third parties. Unauthorized third parties who have not received the encryption/decryption algorithm may not be able to obtain the first security key 31 even if they intercept the second security key 32. Therefore, the second security key 32 may be transmitted to the sensor electronics unit 120 even before the pairing between the analyte monitoring device 100 and the user terminal 200 is completed.

In yet another embodiment, it is also possible for a server to perform verification of the third-party app 21. For example, the whitelist 34 may be stored on the server. The server may receive the second security key 32 from the third-party app 21. Then, the server may determine whether to maintain the communication connection between the sensor electronics unit 120 and the user terminal 200 by determining whether the second security key 32 matches a key stored in the whitelist 34.

Meanwhile, the second security key 32 may include additional information besides the information for verifying the third-party app 21. In one embodiment, the second security key 32 may include information about the third-party app 21. Specifically, the information about the third-party app 21 may include identification information of the third-party app 21 and/or the third party that developed the third-party app 21. Additionally, the information about the third-party app 21 may include information indicating the grade of the third-party app 21. The data provided by the sensor electronics unit 120 to the third-party app 21 or the communication protocol between the sensor electronics unit 120 and the third-party app 21 may vary according to the information about the third-party app 21.

In another embodiment, the second security key 32 may include information about the user terminal 200. The information about the user terminal 200 may include information about the type of the user terminal 200. For example, the information about the user terminal 200 may indicate whether the user terminal 200 is a smartphone or a dedicated receiver for glucose monitoring. The data provided by the sensor electronics unit 120 to the third-party app 21 or the communication protocol between the sensor electronics unit 120 and the third-party app 21 may vary according to the information about the user terminal 200.

In yet another embodiment, the second security key 32 may include a timestamp indicating when the key was generated. The sensor electronics unit 120 may perform verification of the second security key 32 by comparing the timestamp included in the second security key 32 with the current time. For example, the sensor electronics unit 120 may determine that the verification is successful only when the difference between the current time and the timestamp is within a predetermined time period (e.g., 30 seconds). This can prevent replay attacks where an unauthorized third party attempts to reuse a previously intercepted security key. Additionally, even if an unauthorized third party intercepts the second security key 32, they cannot use it after the predetermined time period has elapsed.

The second security key 32 may also include expiration time information. The sensor electronics unit 120 may automatically invalidate the second security key 32 when the current time exceeds the expiration time. For example, the second security key 32 may be valid for 24 hours from its generation time. After the validity period expires, the third-party app 21 needs to perform re-verification to maintain the communication connection. This periodic re-verification can enhance security by limiting the time window during which a potentially compromised security key could be misused.

The sensor electronics unit 120 may request generation of a new second security key 32 upon detection of specific events. These events may include: elapse of 24 continuous hours of data transmission, detection of potential security threats, operating system updates on the user terminal 200, or version changes of the third-party app 21. When such events occur, the sensor electronics unit 120 may transmit a key renewal request to the user terminal 200, and the third-party app 21 may generate a new second security key 32 in response to the request. This dynamic key renewal mechanism can maintain security even when the operating environment changes or potential security vulnerabilities are detected. Additionally, by renewing the security key based on environmental changes of the user terminal 200 or the third-party app 21, the security level can be maintained in accordance with the latest security requirements.

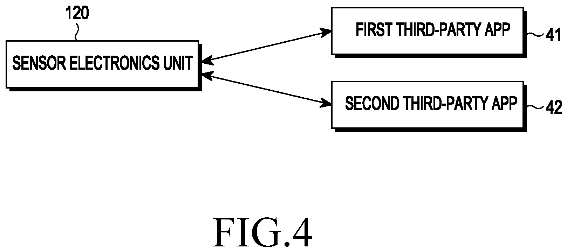

The following description will explain the operation of the sensor electronics unit 120 utilizing the additional information stored in the second security key 32 with reference to FIG. 4.

FIG. 4 is a schematic diagram for explaining operations of the sensor electronics unit according to an embodiment of the present disclosure.

Referring to FIG. 4, the sensor electronics unit 120 may perform verification of the first third-party app 41 and the second third-party app 42. The sensor electronics unit 120 may obtain security keys corresponding to each third-party app during the verification process and identify information about each third-party app included in the security keys. The first third-party app 41 and the second third-party app 42 may be installed on the same user terminal or may be installed on two different user terminals respectively.

When verification of the first third-party app 41 and the second third-party app 42 is successful, the sensor electronics unit 120 may provide data about the user's glucose to the first third-party app 41 and the second third-party app 42. At this time, the data provided to the first third-party app 41 and the second third-party app 42 may be different. For example, glucose signals may be provided to the first third-party app 41, while blood glucose values calculated based on the glucose signals may be provided to the second third-party app 42.

Additionally, the number of data items provided to the first third-party app 41 and the second third-party app 42 in one communication session may be different. For example, the sensor electronics unit 120 may provide data to the first third-party app 41 and the second third-party app 42 once every 5 minutes. At this time, the sensor electronics unit 120 may provide a first number of data items to the first third-party app 41 and a second number of data items, different from the first number, to the second third-party app 42.

The first communication protocol between the sensor electronics unit 120 and the first third-party app 41 may be different from the second communication protocol between the sensor electronics unit 120 and the second third-party app 42. For example, the sensor electronics unit 120 may communicate with the first third-party app 41 once every 5 minutes according to the first communication protocol. And, the sensor electronics unit 120 may communicate with the second third-party app 42 once every minute according to the second communication protocol.

In another embodiment, the sensor electronics unit 120 may control the communication protocol differently based on assigned access levels of the third-party apps. The access levels may be determined based on predetermined technical requirements and security protocols.

When the first third-party app 41 is assigned a first access level, the sensor electronics unit 120 may transmit data at one-minute intervals and provide access to raw sensor signals. At the first access level, the sensor electronics unit 120 may enable real-time data streaming and allow access to historical sensor data. The first access level may be assigned to third-party apps that demonstrate high-level security compliance and validated authentication protocols.

When the second third-party app 42 is assigned a second access level, the sensor electronics unit 120 may transmit data at five-minute intervals and provide only calculated glucose values. At the second access level, the sensor electronics unit 120 may enable periodic data updates and allow access to limited historical data, such as the last 24 hours of glucose measurements. The second access level may be suitable for third-party apps that meet standard security requirements while maintaining moderate system resource usage.

In some cases, the sensor electronics unit 120 may assign a third access level to a third-party app, wherein the sensor electronics unit 120 transmits data at fifteen-minute intervals and provides only averaged glucose values. At the third access level, the sensor electronics unit 120 may enable only manual data updates and allow access to summary data only. This access level may be appropriate for third-party apps with basic data processing capabilities or limited security protocols.

The sensor electronics unit 120 may assign these access levels based on various technical factors. For example, the technical factors may include the third-party app's data processing capabilities, demonstrated security compliance level, validated authentication protocols, and system resource requirements. The assignment of access levels can optimize the use of system resources while maintaining appropriate data access control for each third-party app.

The sensor electronics unit 120 may operate differently depending on the user terminal 200 on which the third-party app is installed. For example, operations for the first third-party app 41 installed on a smartphone may be different from operations for the first third-party app 41 installed on a dedicated receiver. Specifically, the type and number of data provided to the smartphone and the dedicated receiver may be different. Also, the communication protocol between the sensor electronics unit 120 and the smartphone may be different from the communication protocol between the sensor electronics unit 120 and the dedicated receiver.

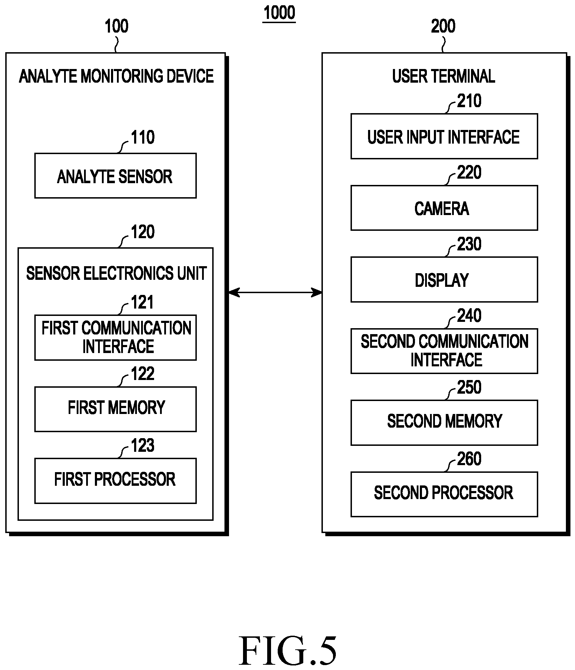

FIG. 5 is a block diagram illustrating a configuration of the analyte monitoring system according to an embodiment of the present disclosure.

Referring to FIG. 5, the analyte monitoring device 100 may include an analyte sensor 110 and a sensor electronics unit 120. The analyte sensor 110 may be a configuration for sensing an analyte signal (or sensor signal). The analyte sensor 110 may include a sensor probe at least a portion of which is inserted into the body. A sensing area that reacts with glucose in the body to measure blood glucose may be formed on the sensor probe.

The sensor electronics unit 120 may perform verification of the analyte monitoring app installed on the user terminal 200. When verification is successful, the sensor electronics unit 120 may transmit to the user terminal 200 either the sensor signal acquired through the analyte sensor 110 or concentration values derived from the sensor signal. Meanwhile, the sensor electronics unit 120 may be referred to as a transmitter.

The sensor electronics unit 120 may include a first communication interface 121, a first memory 122, and a first processor 123. The first communication interface 121 may include at least one communication circuit. The sensor electronics unit 120 may perform communication with the user terminal 200 through the first communication interface 121. The first communication interface 121 may include a Bluetooth module, a Bluetooth Low Energy module, an RF module, and an NFC module.

The first memory 122 may store an operating system (OS) for controlling the overall operation of the components of the sensor electronics unit 120, and instructions or data related to the components of the sensor electronics unit 120. The first memory 122 may be implemented as non-volatile memory (e.g., hard disk, SSD (Solid State Drive), flash memory) or volatile memory. The first memory 122 may store a whitelist including keys corresponding to authorized third parties or third-party apps.

The first processor 123 may be electrically connected to the first memory 122 and control the overall functions and operations of the sensor electronics unit 120. The first processor 123 may control the sensor electronics unit 120 by executing instructions stored in the first memory 122.

The first processor 123 may perform communication connection with the user terminal 200 through the first communication interface 121. The communication connection may include Bluetooth pairing.

The first processor 123 may transmit information about the sensor electronics unit 120 to the user terminal 200 through the first communication interface 121.

The first processor 123 may receive the second security key through the first communication interface 121. The second security key may be generated based on the information about the sensor electronics unit 120 and the first security key. The first security key may be assigned to an application (app) authorized by the manufacturer of the analyte monitoring device 100.

The first processor 123 may perform verification of the second security key. The first processor 123 may perform verification of the second security key by determining whether the second security key matches a key stored in the whitelist.

The first processor 123 may determine whether to maintain the communication connection with the user terminal 200 based on the verification result of the second security key. When verification is successful, the communication connection between the analyte monitoring device 100 and the user terminal 200 may be maintained. Then, the first processor 123 may transmit information about the user's glucose to the user terminal 200 through the first communication interface 121. At this time, the first processor 123 may transmit information about the user's glucose to the user terminal 200 based on characteristics of the user terminal 200 or characteristics of the analyte monitoring app installed on the user terminal 200. Information indicating characteristics of the user terminal 200 or characteristics of the analyte monitoring app installed on the user terminal 200 may be included in the second security key.

When verification fails, the first processor 123 may control the first communication interface 121 to disconnect the connection with the user terminal 200.

The user terminal 200 may provide blood glucose values to the user through the analyte monitoring app. The user terminal 200 may include a user input interface 210, a camera 220, a display 230, a second communication interface 240, a second memory 250, and a second processor 260.

The user input interface 210 may receive user input for communication connection with the analyte monitoring device 100. For example, the user input interface 210 may receive input of an identification number of the analyte monitoring device 100. The user terminal 200 may perform communication connection with the analyte monitoring device 100 based on the identification number of the analyte monitoring device 100. The user input interface 210 may be implemented as a touch screen or buttons.

The camera 220 may capture images for communication connection with the analyte monitoring device 100. For example, the camera 220 may scan a QR code corresponding to the identification number of the analyte monitoring device 100 based on user input. The user terminal 200 may perform communication connection with the analyte monitoring device 100 based on the captured QR code.

The display 230 may display blood glucose values. Additionally, the display 230 may display a message indicating the verification result of the analyte monitoring app or third-party app installed on the user terminal 200.

The second communication interface 240 may include at least one communication circuit. The user terminal 200 may perform communication with the analyte monitoring device 100 through the second communication interface 240. The second communication interface 240 may include a Bluetooth module, a Bluetooth Low Energy module, an RF module, and an NFC module.

The second memory 250 may store an operating system (OS) for controlling the overall operation of the components of the user terminal 200, and instructions or data related to the components of the user terminal 200. The second memory 250 may be implemented as non-volatile memory (e.g., hard disk, SSD (Solid State Drive), flash memory) or volatile memory.

When the analyte monitoring app is an app authorized by the manufacturer of the analyte monitoring device 100, the second memory 250 may store the first security key provided by the manufacturer of the analyte monitoring device 100.

The second processor 260 may be electrically connected to the second memory 250 and control the overall functions and operations of the user terminal 200. The second processor 260 may control the user terminal 200 by executing instructions stored in the second memory 250.

The second processor 260 may receive information about the sensor electronics unit 120 from the analyte monitoring device 100 through the second communication interface 240. The second processor 260 may generate the second security key based on the information about the sensor electronics unit 120 and the first security key. The second processor 260 may control the second communication interface 240 to transmit the second security key to the analyte monitoring device 100.

The second processor 260 may control the display 230 to display a message indicating the verification result of the analyte monitoring app or third-party app. For example, when verification fails, the display 230 may display an error message.

Various exemplary embodiments of the present disclosure described above may be implemented in a computer or a computer readable recording medium using software, hardware, or a combination of software and hardware. **In** some cases, embodiments described in the present disclosure may be implemented by the processor itself. According to a software implementation, embodiments such as procedures and functions described in the present disclosure may be implemented by separate software modules. Each of the software modules may perform one or more functions and operations described in the present disclosure.

Computer instructions for performing processing operations according to the diverse embodiments of the present disclosure described above may be stored in a non-transitory computer-readable medium. The computer instructions stored in the non-transitory computer-readable medium allow a specific machine to perform the processing operations according to the diverse embodiments described above when they are executed by a processor.

The non-transitory computer-readable medium is not a medium that stores data for a while, such as a register, a cache, a memory, or the like, but means a medium that semi-permanently stores data and is readable by the apparatus. A specific example of the non-transitory computer-readable medium may include a compact disk (CD), a digital versatile disk (DVD), a hard disk, a Blu-ray disk, a universal serial bus (USB), a memory card, a read only memory (ROM), or the like.

The machine-readable storage medium may be provided in the form of a non-transitory storage medium. Here, the "non-transitory storage medium" means that the storage medium is a tangible device, and does not include a signal (for example, electromagnetic waves), and the term does not distinguish between the case where data is stored semi-permanently on a storage medium and the case where data is temporarily stored thereon. For example, the "non-transitory storage medium" may include a buffer in which data is temporarily stored.

The methods according to the diverse embodiments disclosed in the document may be included and provided in a computer program product. The computer program product may be traded as a product between a seller and a purchaser. The computer program product may be distributed in the form of a machine-readable storage medium (for example, compact disc read only memory (CD-ROM)), or may be distributed (for example, download or upload) through an application store (for example, Play Store^{™}) or may be directly distributed (for example, download or upload) between two user devices (for example, smart phones) online. In a case of the online distribution, at least some of the computer program products (for example, downloadable app) may be at least temporarily stored in a machine-readable storage medium such as a memory of a server of a manufacturer, a server of an application store, or a relay server or be temporarily created.

According to an embodiment of the present disclosure, verification of the analyte monitoring app may be performed, and a communication connection may be maintained with the verified analyte monitoring app. Therefore, glucose signals may be provided only to the verified analyte monitoring app. Accordingly, this may prevent unverified analyte monitoring apps from providing blood glucose information to the user.

In addition, effects obtainable or predicted by the embodiments of the present disclosure have been disclosed directly or implicitly in the detailed description of the embodiments of the present disclosure. For example, various effects predicted according to embodiments of the present disclosure have been disclosed in the above-described detailed description.

Other aspects, advantages and prominent features of the present disclosure will become apparent to those skilled in the art from the above detailed description which disclosed various embodiments of the present disclosure taken in conjunction with the accompanying drawings.

Although embodiments of the present disclosure have been illustrated and described hereinabove, the present disclosure is not limited to the above-described specific embodiments, but may be variously modified by those skilled in the art to which the present disclosure pertains without departing from the gist of the present disclosure as disclosed in the accompanying claims. These modifications should also be understood to fall within the scope and spirit of the present disclosure.

## Claims

1. An analyte monitoring device, comprising:
an analyte sensor configured to be at least partially inserted into a body of a user to detect a signal related to glucose concentration; and
a sensor electronics unit electrically connected to the analyte sensor to acquire the signal, wherein the sensor electronics unit is configured to:
perform communication connection with a user terminal,
transmit information about the sensor electronics unit to the user terminal,
receive, from the user terminal, a second security key generated based on the information about the sensor electronics unit and a first security key,
perform verification of the second security key, and
determine whether to maintain the communication connection based on the verification result.

2. The analyte monitoring device of claim 1, wherein the first security key is assigned to an application authorized by a manufacturer of the analyte monitoring device, and wherein the second security key is used for verification of the application.

3. The analyte monitoring device of claim 2, wherein the second security key includes information indicating characteristics of the user terminal or characteristics of the application, and wherein the sensor electronics unit is configured to operate based on the characteristics of the user terminal or the characteristics of the application when the verification is successful.

4. The analyte monitoring device of claim 2, wherein the application is a third-party application.

5. The analyte monitoring device of claim 1, wherein the communication connection is established through Bluetooth pairing, wherein the information about the sensor electronics unit is transmitted in an unencrypted state before the Bluetooth pairing, and wherein the second security key is received in an encrypted state after the Bluetooth pairing.

6. The analyte monitoring device of claim 1, wherein the communication connection is performed based on at least one of Bluetooth and Bluetooth Low Energy (BLE) connection methods.

7. The analyte monitoring device of claim 1, wherein the sensor electronics unit performs verification of the second security key by determining whether the second security key matches a pre-stored key.

8. The analyte monitoring device of claim 7, wherein the sensor electronics unit: obtains a new key by decrypting the second security key based on a predetermined algorithm, and determines whether the new key matches the pre-stored key.

9. The analyte monitoring device of claim 7, wherein the sensor electronics unit stores a whitelist including a plurality of security keys assigned to a plurality of third parties, including the pre-stored key.

10. The analyte monitoring device of claim 1, wherein the sensor electronics unit: maintains the communication connection when the verification is successful, and disconnects the communication connection when the verification fails.

11. A method for controlling an analyte monitoring device comprising an analyte sensor configured to be at least partially inserted into a body of a user to detect a signal related to glucose concentration and a sensor electronics unit electrically connected to the analyte sensor to acquire the signal, the method comprising:
performing communication connection with a user terminal;
transmitting information about the sensor electronics unit to the user terminal;
receiving, from the user terminal, a second security key generated based on the information about the sensor electronics unit and a first security key;
performing verification of the second security key; and
determining whether to maintain the communication connection based on the verification result.

12. The method of claim 11, wherein the first security key is assigned to an application authorized by a manufacturer of the analyte monitoring device, and wherein the second security key is used for verification of the application.

13. The method of claim 12, wherein the second security key includes information indicating characteristics of the user terminal or characteristics of the application, and wherein the method further comprises operating based on the characteristics of the user terminal or the characteristics of the application when the verification is successful.

14. The method of claim 12, wherein the application is a third-party application.

15. The method of claim 11, wherein the communication connection is established through Bluetooth pairing, wherein the information about the sensor electronics unit is transmitted in an unencrypted state before the Bluetooth pairing, and wherein the second security key is received in an encrypted state after the Bluetooth pairing.

16. The method of claim 11, wherein the communication connection is performed based on at least one of Bluetooth and Bluetooth Low Energy (BLE) connection methods.

17. The method of claim 11, wherein the sensor electronics unit performs verification of the second security key by determining whether the second security key matches a pre-stored key.

18. The method of claim 17, wherein performing verification of the second security key comprises: obtaining a new key by decrypting the second security key based on a predetermined algorithm; and determining whether the new key matches the pre-stored key.

19. The method of claim 17, wherein the sensor electronics unit stores a whitelist including a plurality of security keys assigned to a plurality of third parties, including the pre-stored key.

20. The method of claim 11, further comprising: maintaining the communication connection when the verification is successful; and disconnecting the communication connection when the verification fails.
